# EUROPEAN PATENT APPLICATION

(11) **EP 4 467 149 A1**
(43) Date of publication of application: **27.11.2024**
(21) Application number: 23743461.8
(22) Date of filing: 18.01.2023
(51) Int. Cl.: A61K 35/747, A61K 35/745, A61K 35/744, A61P 39/02, A23L 33/135, A61K 8/99, A61Q 19/00

(54) **COMPOSITION COMPRISING PROBIOTICS FOR REMOVAL OF HEAVY METALS**

(30) Priority: 18.01.2022 KR 20220007383
(71) Applicant: Cell Biotech Co., Ltd., Wolgot-myeon Gyeonggi-do 10003 (KR)
(72) Inventor: CHUNG, Myung Jun, Seoul 06622 (KR)
(74) Representative: Stöckeler, Ferdinand
(86) International application number: PCT/KR2023/000863
(87) International publication number: WO 2023/140614

(57) **Abstract**

The present invention relates to a composition, pharmaceutical composition, food composition or cosmetic composition, for removing heavy metals, each composition containing one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.* The composition for removing the heavy metals according to the present invention has the advantage of being effectively used to remove heavy metals by expelling them in vivo, due to the composition being excellent for tolerance to heavy metals, antibacterial effect, and heavy metal expulsion.

## Description

### [Technical Field]

The present invention relates to a probiotics-containing composition for removing heavy metals.

### [Background Art]

With the development of society, technology, and civilization, the severity of environmental pollution has been caused by various causes. Among these, heavy metals discharged during production activities pose a high risk to the human body and at the same time threaten the ecosystem. Our human body, which can be easily exposed to these risks, is exposed to the surrounding environment such as food, water, air, and soil, and always intakes heavy metals, although there may be some differences from person to person.

In particular, the intake of harmful heavy metals that Koreans consume through food is close to the acceptable daily intake (ADI), which is the allowable daily intake for the human body, and once these heavy metals enter the human body, they are not easily discharged. Among these, toxic heavy metals react with human tissue even at somewhat low concentrations and slowly become toxic. Thus, when poisoning exceeds a certain level, recovery and complete cure are impossible, which is a major problem in the modern food society.

Among heavy metals that are toxic to the human body, cadmium (Cd) exists in relatively low concentrations in nature, but is very harmful and its use in various factories and industrial sites is increasing. Thus, as a result of evaluating its health impact on the human body, interest in it as an environmental pollutant has increased as it causes acute and chronic poisoning. Research results have reported that probiotics reduce the absorption rate of cadmium in the body, which causes serious damage to the human body.

It is generally known that the absorption of heavy metals by microorganisms occurs in two ways. The first way is adsorption on a cell surface by binding of cations to anion groups present on the cell surface, and the second way is a process of accumulation within cells through metabolism, and currently, research is mainly conducted on cell surface adsorption.

Research on the heavy metal expulsion effect of lactic acid bacteria is currently insufficient. Therefore, the present inventors have selected strains that are excellent for heavy metal expulsion, based on acid resistance to probiotics and functionality to bile acid resistance, and have completed the present invention.

### [Disclosure]

### [Technical Problem]

The present inventors made research efforts to develop probiotic strains that may be applied to heavy metal adsorption or removal.

The present invention aims to provide a composition for removing heavy metals, the composition containing one or more lactic acid bacteria selected from the group consisting of bacteria of the genus *Lactobacillus,* bacteria of the genus *Bifidobacterium,* bacteria of the genus Enterococcus, bacteria of the genus Pediococcus, and bacteria of the genus Streptococcus.

The present invention also aims to provide a cosmetic composition for removing heavy metals, the cosmetic composition containing one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.*

The present invention also aims to provide a pharmaceutical composition for removing heavy metals, the pharmaceutical composition containing one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.*

The present invention also aims to provide a food composition for removing heavy metals, the food composition containing one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.*

Other objectives and technical features of the present invention are presented in more detail by the following description, claims, and drawings.

### [Technical Solution]

An aspect of the present invention relates to a composition for removing heavy metals, the composition containing one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.*

In the present invention, the lactic acid bacteria are one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.*

The lactic acid bacteria of the present invention may be probiotic lactic acid bacteria in the sense that, when the lactic acid bacteria are administered to a body, the lactic acid bacteria settle in a gut and work in a beneficial synergistic manner with gut microbiota.

According to an embodiment of the present invention, lactic acid bacteria of the genus Lactobacillus may be one or more lactic acid bacteria selected from the group consisting of *Lactobacillus acidophilus, Lactobacillus gasseri, Lactobacillus lactis, Lactobacillus paracasei, Lactobacillus helveticus, Lactobacillus reuteri, Lactobacillus rhamnosus, Lactobacillus casei, Lactobacillus fermentum,* and *Lactobacillus plantarum.*

According to an embodiment of the present invention, lactic acid bacteria of the genus *Bifidobacterium* may be one or more lactic acid bacteria selected from the group consisting of *Bifidobacterium infantis, Bifidobacterium bifidum, Bifidobacterium lactis, Bifidobacterium longum,* and *Bifidobacterium breve.*

According to an embodiment of the present invention, bacteria of the genus *Enterococcus* may be one or more lactic acid bacteria selected from the group consisting of *Enterococcus faecium* and *Enterococcus faecalis.*

According to an embodiment of the present invention, bacteria of the genus *Pediococcus* may be lactic acid bacteria that are *Pediococcus pentosaceus.*

According to an embodiment of the present invention, bacteria of the genus *Streptococcus* may be lactic acid bacteria that are *Streptococcus thermophilus.*

The composition for removing the heavy metals in the present invention may have a tolerance to heavy metals. For example, the tolerance may have one or more properties selected from minimum inhibitory concentrations (MICs), bacterial growth, and heavy metal removal or binding capacity.

The composition for removing the heavy metals in the present invention serves to promote expulsion of heavy metals. Therefore, the composition for removing the heavy metals in the present invention serves to expel heavy metals accumulated in blood and tissues to the outside of a body.

The heavy metals in the present invention may be cationic heavy metals or anionic heavy metals. For example, the cationic heavy metals may be cadmium (Cd), copper (Cu), and lead (Pb), or zinc (Zn), and the anionic heavy metals may be arsenic (As), chromium (Cr), selenium (Se), or antimony (Sb).

The cationic heavy metals in the present invention are cadmium, and the bacteria may have a cadmium removal capacity of 22 % or more in 100 mg/L of a cadmium solution. Preferably, the bacteria may have a cadmium removal capacity of 29 % or more in 50 mg/L of a cadmium solution. More preferably, the bacteria may have a cadmium removal capacity of 51 % or more in 5 mg/L of a cadmium solution.

The cationic heavy metals in the present invention are lead, and the bacteria may have a lead removal capacity of 22 % or more in 100 mg/L of a lead solution. Preferably, the bacteria may have a lead removal capacity of 41 % or more in 50 mg/L of a lead solution. More preferably, the bacteria may have a lead removal capacity of 76 % or more in 5 mg/L of a lead solution.

Another aspect of the present invention provides a pharmaceutical composition for preventing or treating heavy metal poisoning, the pharmaceutical composition including one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.*

Regarding the pharmaceutical composition of the present invention, the bacteria of the genus *Lactobacillus,* the bacteria of the genus *Bifidobacterium,* the bacteria of the genus *Enterococcus,* the bacteria of the genus *Pediococcus,* and the bacteria of the genus *Streptococcus* are as described in connection with the above-described "composition for removing heavy metals," which is another aspect of the present invention, and thus, redundant descriptions thereof are not provided.

In the present invention, the term "preventing" means inhibiting development of an illness, disease, or symptom in an animal that has not been diagnosed as having the illness, disease, or symptom due to the illness, but is predisposed to contracting such an illness, disease, or symptom.

In the present invention, the term "treating" means (i) inhibiting development of an illness, disease, or symptom, (ii) alleviating an illness, disease, or symptom, or (iii) removing an illness, disease, or symptom.

Pharmaceutically acceptable carriers included in the pharmaceutical composition of the present invention include carbohydrate compounds (for example, lactose, amylose, dextrose, sucrose, sorbitol, mannitol, starch, cellulose, etc.), gum acacia, calcium phosphate, alginate, gelatin, calcium silicate, microcrystalline cellulose, polyvinylpyrrolidone, cellulose, water, syrup, salt solutions, alcohol, gum arabic, vegetable oils (for example, corn oil, cottonseed oil, soy milk, olive oil, coconut oil), polyethylene glycol, methyl cellulose, methyl hydroxy benzoate, propylhydroxybenzoate, talc, magnesium stearate, and mineral oil, which are commonly used during pharmaceutical formulation, but are not limited thereto.

The pharmaceutical composition of the present invention may further include, in addition to the above-described ingredients, a lubricant, a humectant, a sweetener, a flavoring agent, an emulsifier, a suspending agent, a preservative, etc. Suitable pharmaceutically acceptable carriers and formulations are described in detail in Remington's Pharmaceutical Sciences (19th ed., 1995).

The appropriate dosage of the pharmaceutical composition of the present invention varies depending on factors, such as formulation method, administration method, patient's age, body weight, sex, pathological condition, food, administration time, administration route, excretion rate, and reaction sensitivity. Meanwhile, the oral dosage of the pharmaceutical composition of the present invention is preferably 0.001-1,000 mg/kg (body weight) per day.

The pharmaceutical composition of the present invention may be prepared in a unit dose form by formulating a pharmaceutically acceptable carrier and/or excipient, or may be prepared by placing the pharmaceutically acceptable carrier and/or excipient in a multi-dose container, according to a method that may be easily performed by a person skilled in the art. At this time, a dosage form may be a solution, suspension or emulsion in an oil or aqueous medium, or may be an extract, powder, granules, a tablet, or a capsule, and may further include dispersants or stabilizers.

Another aspect of the present invention provides a food composition including one or more lactic acid bacteria selected from the group consisting of bacteria of the genus Lactobacillus, bacteria of the genus Bifidobacterium, bacteria of the genus Enterococcus, bacteria of the genus Pediococcus, and bacteria of the genus Streptococcus.

Regarding the food composition of the present invention, the bacteria of the genus Lactobacillus, the bacteria of the genus Bifidobacterium, the bacteria of the genus Enterococcus, the bacteria of the genus Pediococcus, and the bacteria of the genus Streptococcus are as described in connection with the above-described "composition for removing heavy metals," which is another aspect of the present invention, and thus, redundant descriptions thereof are not provided.

The food composition of the present invention may be prepared as functional food, nutritional supplements, health food, or food additives, but is not limited thereto.

The food composition of the present invention may be prepared in various forms according to conventional methods known in the art, for example, in the form of a beverage such as fermented milk, or in the form of powder.

The food composition of the present invention includes not only the above-described active ingredients of the present invention, but also ingredients commonly added during food production, and for example, includes proteins, carbohydrates, fats, nutrients, a seasoning, and a flavoring agent. Examples of the above-described carbohydrates include common sugars, such as: monosaccharides, such as glucose, fructose, etc.; disaccharides, such as maltose, sucrose, oligosaccharides, etc.; and polysaccharides, such as dextrin, cyclodextrin, etc., and sugar alcohols, such as xylitol, sorbitol, erythritol, etc. As the flavoring agent, natural flavoring agents (thaumatin, stevia extract (for example, rebaudioside A, glycyrrhizin, etc.)) and synthetic flavoring agents (saccharin, aspartame, etc.) may be used.

When the food composition of the present invention is prepared in the form of a beverage, in addition to the above-described active ingredients of the present invention, citric acid, high fructose corn syrup, sugar, glucose, acetic acid, malic acid, fruit juice, eucommia extract, jujube extract, licorice extract, etc. may be additionally included.

Another aspect of the present invention provides a cosmetic composition including one or more lactic acid bacteria selected from the group consisting of bacteria of the genus Lactobacillus, bacteria of the genus Bifidobacterium, bacteria of the genus Enterococcus, bacteria of the genus Pediococcus, and bacteria of the genus Streptococcus.

Regarding the cosmetic composition of the present invention, the bacteria of the genus Lactobacillus, the bacteria of the genus Bifidobacterium, the bacteria of the genus Enterococcus, the bacteria of the genus Pediococcus, and the bacteria of the genus Streptococcus are as described in connection with the above-described "composition for removing heavy metals," which is another aspect of the present invention, and thus, redundant descriptions thereof are not provided.

Ingredients included in the cosmetic composition of the present invention are active ingredients and may include, in addition to the active ingredients, ingredients commonly used in cosmetic compositions, and may include, for example, a conventional supplement, such as an antioxidant, a stabilizer, a solubilizer, a vitamin, a pigment, and an odoriferous substance, and a carrier.

The cosmetic composition of the present invention may be prepared in any dosage form commonly prepared in the art, and may be used, for example, as a solution, a suspension, an emulsion, paste, gel, cream, lotion, powder, soap, surfactant-containing cleansing agent, oil, powder foundation, emulsion foundation, wax foundation, a pack, massage cream, and a spray, but is not limited thereto. More specifically, the cosmetic composition may be prepared in the form of softening lotion, nourishing lotion, nourishing cream, massage cream, essence, eye cream, cleansing cream, cleansing foam, cleansing water, a pack, a spray, or powder.

When the cosmetic composition of the present invention is paste, cream, or gel, animal oil, vegetable oil, wax, paraffin, starch, tracant, cellulose derivatives, polyethylene glycol, silicone, bentonite, silica, talc, or zinc oxide may be used as a carrier ingredient.

When the cosmetic composition of the present invention is a solution or an emulsion, a solvent, a solubilizer, or an emulsifier is used as a carrier ingredient, and examples thereof are water, ethanol, isopropanol, ethyl carbonate, ethyl acetate, benzyl alcohol, benzyl benzoate, propylene glycol, 1,3-butyl glycol oil, glycerol aliphatic esters, polyethylene glycol, or fatty acid esters of sorbitan.

When the cosmetic composition of the present invention is a suspension, a liquid diluent, such as water, ethanol, or propylene glycol, a suspending agent, such as ethoxylated isostearyl alcohol, polyoxyethylene sorbitol ester, and polyoxyethylene sorbitan ester, microcrystalline cellulose, aluminum metahydroxide, bentonite, agar, or tracant may be used as a carrier ingredient.

When the cosmetic composition of the present invention is powder or a spray, lactose, talc, silica, aluminum hydroxide, calcium silicate, or polyamide powder may be used as a carrier ingredient, and in particular, when the cosmetic composition of the present invention is a spray, a propellant, such as chlorofluorohydrocarbon, propane/butane, or dimethyl ether, may be further included.

When the cosmetic composition of the present invention is a surfactant-containing cleansing agent, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamidobetaine, fatty alcohol, fatty acid glyceride, fatty acid diethanolamide, vegetable oil, lanolin derivatives, or ethoxylated glycerol fatty acid esters may be used as a carrier ingredient.

When the cosmetic composition of the present invention is a soap, a surfactant-containing cleansing agent, or a surfactant-free cleansing agent, the cosmetic composition may be applied to the skin and then wiped off, removed, or washed with water. Examples of the soap include liquid soap, powdered soap, solid soap, and oil soap, examples of the surfactant-containing cleansing agent include cleansing foam, cleansing water, cleansing towel, and cleansing pack, and examples of the surfactant-free cleansing agent include cleansing cream, cleansing lotion, cleansing water, and cleansing gel, but the examples are not limited thereto.

### [Advantageous Effects]

The present invention relates to a composition, pharmaceutical composition, food composition or pharmaceutical composition, for removing heavy metals, each composition containing one or more lactic acid bacteria selected from the group consisting of bacteria of the genus *Lactobacillus,* bacteria of the genus *Bifidobacterium,* bacteria of the genus *Enterococcus,* bacteria of the genus *Pediococcus,* and bacteria of the genus *Streptococcus.* The composition for removing the heavy metals according to the present invention may be effectively used to remove heavy metals by expelling them in vivo, due to the composition being excellent for tolerance to heavy metals, antibacterial effect, and heavy metal expulsion.

### [Description of Drawings]

FIG. 1A shows results of cadmium (Cd) MIC tests and growth curves of LA1 and LGA.
FIG. 1B shows results of cadmium (Cd) MIC tests and growth curves of EF4 and EFL2.
FIG. 1C shows results of cadmium (Cd) MIC tests and growth curves of SL6 and LPC5.
FIG.1D shows results of cadmium (Cd) MIC tests and growth curves of LH5 and LU4.
FIG. 1E shows results of cadmium (Cd) MIC tests and growth curves of LR5 and LC5.
FIG.1F shows results of cadmium (Cd) MIC tests and growth curves of PP1, SL4 and LF2.
FIG. 1G shows results of cadmium (Cd) MIC tests and growth curves of LP3 and BT1.
FIG. 1H shows results of cadmium (Cd) MIC tests and growth curves of BF3 and BL3.
FIG. 1I shows results of cadmium (Cd) MIC tests and growth curves of BG7 and BR3 .
FIG.1J shows results of a cadmium (Cd) MIC test and growth curve of ST3.
FIG.2 shows a cadmium (Cd) removal capacity (%) and p-value of each individual strain in 50 mg/L of a cadmium solution.
FIG.3 shows a cadmium (Cd) removal capacity (%) and p-value of each individual strain in 5 mg/L of a cadmium solution.
FIG.4 shows a cadmium (Cd) removal capacity (%) and p-value of each individual strain in 100 mg/L of a cadmium solution.
FIG.5A shows results of lead (Pb) MIC tests and growth of LA1 and 11 other strains.
FIG.5B shows results of lead (Pb) MIC tests and growth of LP3 and 6 other strains.
FIG.5C shows a graph of changes in lead (Pb) concentration of LA1 and 10 other strains.
FIG.5D shows a graph of changes in lead (Pb) concentration of BL3 and 7 other strains.
FIG.6 shows a lead (Pb) removal capacity (%) of each individual strain in 50 mg/L of a lead solution.
FIG.7 shows a lead (Pb) removal capacity (%) of each individual strain in 5 mg/L of a lead solution.
FIG.8 shows a lead (Pb) removal capacity (%) of each individual strain in 100 mg/L of a lead solution.
FIG.9 shows SEM images of samples obtained by administering *Lactobacillus plantarum* and *Lactobacillus acidophilus* to 100 mg/L of a cadmium solution and 100 mg/L of a lead solution.
FIG. 10 is a schematic diagram showing a process of selecting strains with excellent cadmium removal capacity.

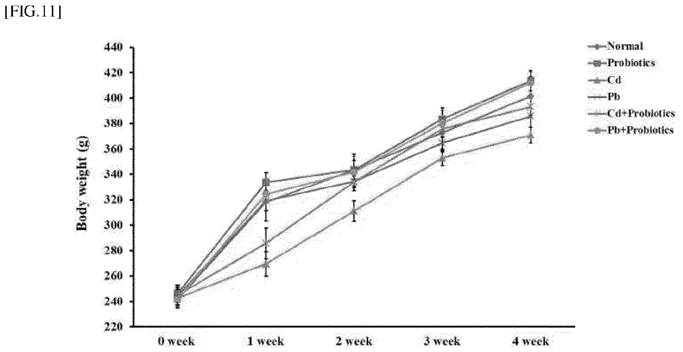

FIG. 11 shows changes in body weight and liver weight caused by probiotics of the present invention.
FIG. 12 shows changes in excretion of cadmium (Cd) and lead (Pb) into feces by probiotics of the present invention.
FIG. 13 shows changes in the amount of cadmium (Cd) and lead (Pb) in blood and tissues (liver, kidney) by probiotics of the present invention.

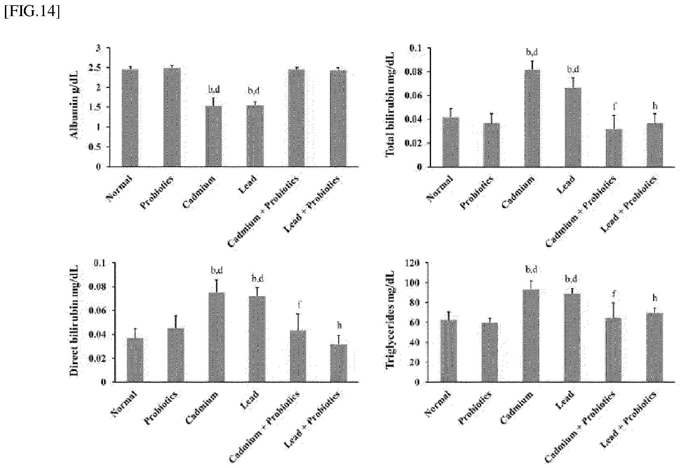

FIG. 14 shows hematological changes of cadmium (Cd) and lead (Pb) by probiotics of the present invention.

### [Mode for Invention]

Preferred embodiments of the present invention are described. However, the embodiments of the present invention may be modified into various other forms, and the scope of the present invention is not limited to the embodiments described below. In addition, the embodiments of the present invention are provided to more completely explain the present invention to those with average knowledge in the relevant technical field.

### Experimental method

### Experimental Example 1. Selection of probiotics with heavy metal removal capacity

In order to select probiotic strains with the capacity to adsorb or remove heavy metals, screening (in vitro) was performed.

Bacteria of the genus *Lactobacillus,* bacteria of the genus *Bifidobacterium,* bacteria of the genus *Enterococcus,* bacteria of the genus *Pediococcus,* and bacteria of the genus *Streptococcus* were set as probiotic strains, and *Lactobacillus acidophilus, Lactobacillus gasseri, Lactobacillus lactis, Lactobacillus paracasei, Lactobacillus helveticus, Lactobacillus reuteri, Lactobacillus rhamnosus, Lactobacillus casei, Lactobacillus fermentum,* and *Lactobacillus plantarum* were set as the bacteria of the genus *Lactobacillus, Bifidobacterium infantis, Bifidobacterium bifidum, Bifidobacterium lactis, Bifidobacterium longum,* and *Bifidobacterium breve* were set as the bacteria of the *genus Bifidobacterium, Enterococcus faecium* and *Enterococcus faecalis* were set as the bacteria of the genus *Enterococcus, Pediococcus pentosaceus* was set as the bacteria of the genus *Pediococcus,* and *Streptococcus thermophilus* was set as the bacteria of the genus *Streptococcus.*

### Experimental Example 2. Measurement of antibacterial effect (MIC Test)

Each individual strain selected in Experimental Example 1 was subj ected to strain selection and efficacy evaluation (in vitro) by using an antibacterial activity test (MIC test) with respect to heavy metals.

First, each strain was seeded on an MRS or BL agar plate by using a sterilized paper disk. For the antibacterial activity test, cadmium (Cd) (concentration range: 50 mg/L to 1,000 mg/L, 50 µl/disk) and lead (Pb) (concentration range: 50 mg/L to 5,000 mg/L, 50 µl/disk) among heavy metals were used to select strains resistant to cadmium or lead. In addition, binding capacity assay was used to select strains with adhesion to cadmium or lead.

### Experimental Example 3. Bacterial growth test

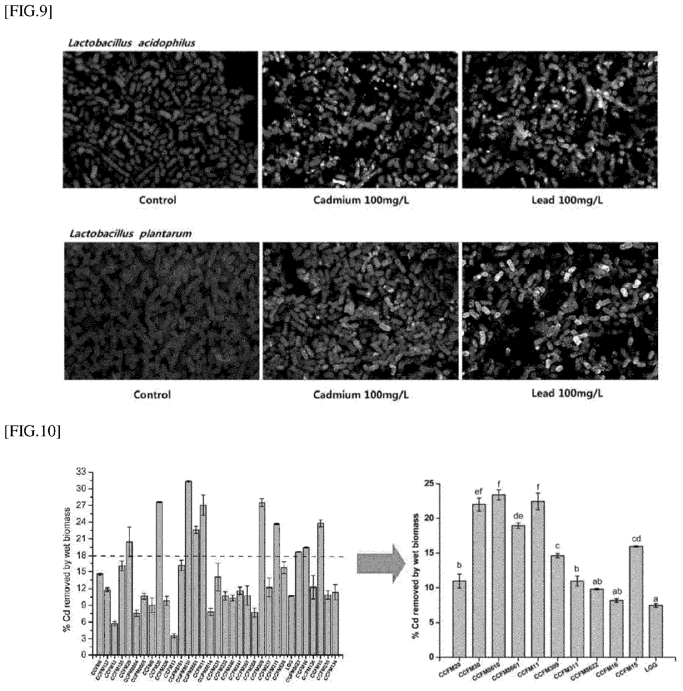

Each individual strain selected in Experimental Example 1 was subj ected to strain selection and efficacy evaluation (in vitro) by using a bacterial growth test with respect to heavy metals. Cadmium and lead were administered to each individual strain, and changes in growth depending on the concentrations of cadmium and lead were measured (see FIG. 10).

0 mg/L, 100 mg/L, 500 mg/L, and 1,000 mg/L, which are concentrations based on results of antibacterial effect evaluation (MIC test) in Experiment Example 2, were set as a cadmium concentration range, and a growth pattern of each individual strain in MRS or BL broth was analyzed according to a set cadmium concentration. 0 mg/L, 50 mg/L, 100 mg/L, 500 mg/L, 1,000 mg/L, 2,000 mg/L, 3,000 mg/L, 4,000 mg/L, and 5,000 mg/L, which are concentrations based on the results of the antibacterial effect evaluation (MIC test) in Experimental Example 2, were set as a cadmium concentration range.

Evaluation values for cadmium and lead refer to re-confirmation of growth of each individual strain in the MRS or BL broth with respect to the results of the antibacterial effect evaluation (MIC test) in Experimental Example 2.

### Experimental Example 4. Measurement of heavy metal removal capacity and binding capacity assay

**The heavy metal removal capacity of each individual strain selected in Experimental Example 1** was measured. First, each individual strain was incubated for 18 hours, and pellets were incubated in 1 mg/L (1 ppm) of cadmium solutions (Cd solution media) at 37 °C for 2 hours. Afterwards, the concentrations of cadmium in the cadmium solutions (Cd solution media) were measured, and metal removal efficiency (MRE) or Cd uptake was measured by using the formula below.
MRE = Ci-Cf / (tf-ti)M (Ci = initial Cd concentration, Cf = final Cd concentration, ti = initial time, tf = final time, M = mass of cell, µg h-1 mg-1)
Cd uptake = (C0-C1)V/m (C0 (mg/L)= initial Cd concentration, C 1 (mg/L) = Cd concentration after adsorption, V(L) = volume of the solution, m(g) = amount of biomass)

In addition, cadmium (Cd) binding characterization and lead (Pb) binding characterization of each individual strain were analyzed.

To analyze cadmium binding characteristics, each individual strain was incubated for 18 hours, pellets were subjected to resuspension by using 50 mg/L of a cadmium (Cd) solution, and then adjusted to pH 6 by using a NaOH or HNO3 solution. Then, after incubation at 37 °C for 1 hour, the cadmium concentrations of supernatants were measured, and strains with excellent cadmium removal and binding capacity were selected. Lead binding characteristic analysis was performed in the same manner as the cadmium binding characteristic analysis to measure and evaluate lead concentrations.

Quantitative analysis of cadmium and lead was measured by using an atomic absorption spectrometer, and cadmium and lead, which were attached to the surface of a finally selected strain, were measured by using a scanning electron microscope (SEM).

### Experimental Example 5. Animal experiment measurement (In vivo test)

The probiotic strains of Experimental Example 1 were evaluated in animal models (in vivo). Experimental animals used were SD rat males, 6 rats/group. Cadmium chloride (CdCl2, Cd), lead acetate (Pb(C2H3O2)2, Pb), and probiotics (LA+LP, Pro) were administered to the rats at concentrations of 10 mg/kg of cadmium, 10 mg/kg of lead, and 1 × 10 9 CFU/rat of probiotics (LA+LP) for a period of 4 weeks, and a specific animal testing schedule is as follows.

**[Table 1]**

| Experimental group | -1 to 0 weeks | 0 week | 1 week | 2 week | 3 week | 4 week |
|---|---|---|---|---|---|---|
| Normal | Acclimation | | | | | |
| Normal+Pro | | Pro 4 week administration | | | | |
| Normal+Cd | | Cd 4 week administration | | | | |
| Normal+Pb | | Pb 4 week administration | | | | |
| Normal+Pro+C d | | Pro + Cd 4 week administration | | | | |
| Normal+Pro+C d | | Pro + Pb 4 week administration | | | | |

The body weight of each experimental group was measured at each week, and the liver and kidney weights of each entity were measured after the administration. During the administration period, each entity's feces were collected every week to measure the concentrations of cadmium and lead in the feces. After the administration was completed, the concentrations of cadmium and lead in blood and tissues (liver, kidney) of each entity were measured. To measure this, an atomic absorption spectrometer was used. In addition, after 4 weeks of administration, hematological analysis was performed to analyze lipid and liver levels of each entity.

### Experiment result

### Example 1. Evaluation (in vitro) of antibacterial activity tests (MIC tests) and bacterial growth by probiotics with respect to cadmium (Cd)

For each individual strain selected in Experimental Example 1, a heavy metal antibacterial activity test (MIC test) and bacterial growth were analyzed, and the tolerance ability is shown in Table 2 below.

**[Table 2]**

| Type of resistant | Strains | Cd MIC (mg/L) |
|---|---|---|
| | *L. acdidophilus* (LA1) | > 1,000 mg/L |
| | *L. gasseri* (LGA) | > 1,000 mg/L |
| | *E. faecium* (EF4) | > 1,000 mg/L |
| | *E. faecalis* (EFL2) | > 1,000 mg/L |
| Cd-resistant | *L. lactis* (SL6) | > 1,000 mg/L |
| | *L. paracasei* (LPC5) | < 600 mg/L |
| | *L. helveticus* (LH5) | < 500 mg/L |
| | *L. reureri* (LU4) | < 300 mg/L |
| | *B. infantis* (BT1) | < 300 mg/L |
| | *L. rhamnosus* (LR5) | < 250 mg/L |
| | *B. bifidum* (BF3) | < 250 mg/L |
| | *L. casei* (LC5) | < 150 mg/L |
| | *P. pentosaceus* (PP1,SL4) | < 100 mg/L |
| | *L. fermentum* (LF2) | < 50 mg/L |
| | *L. plantarum* (LP3) | < 50 mg/L |
| | *B. lactis* (BL3) | < 50 mg/L |
| | *B. longum* (BG7) | < 50 mg/L |
| | *B. breve* (BR3) | < 50 mg/L |
| | *S. thermophilus* (ST3) | < 50 mg/L |

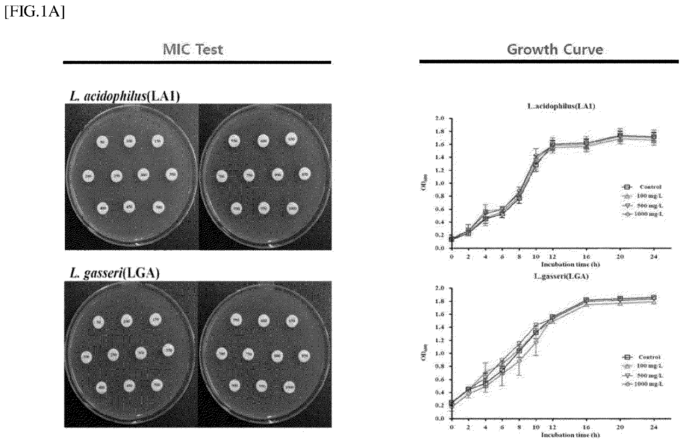

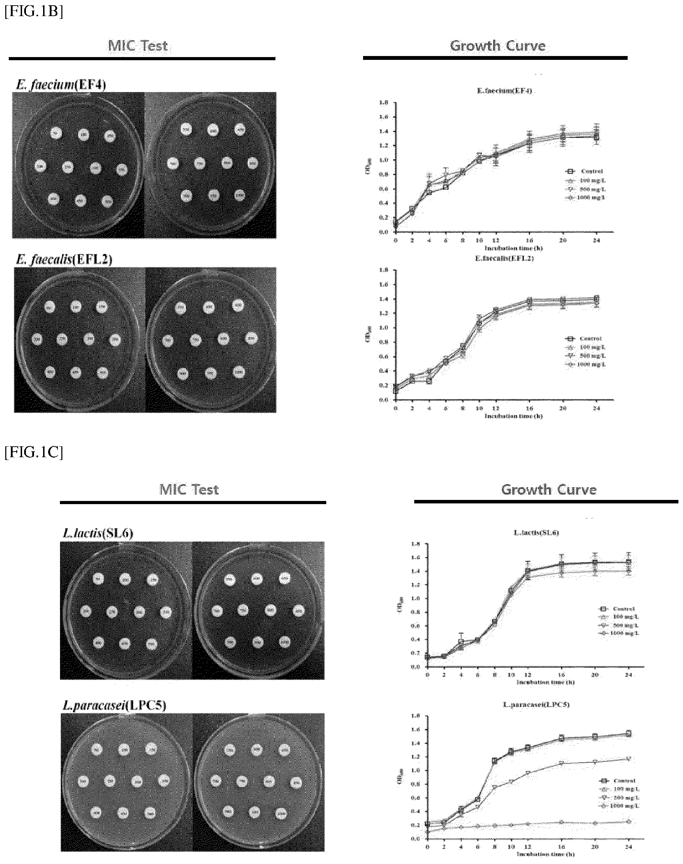

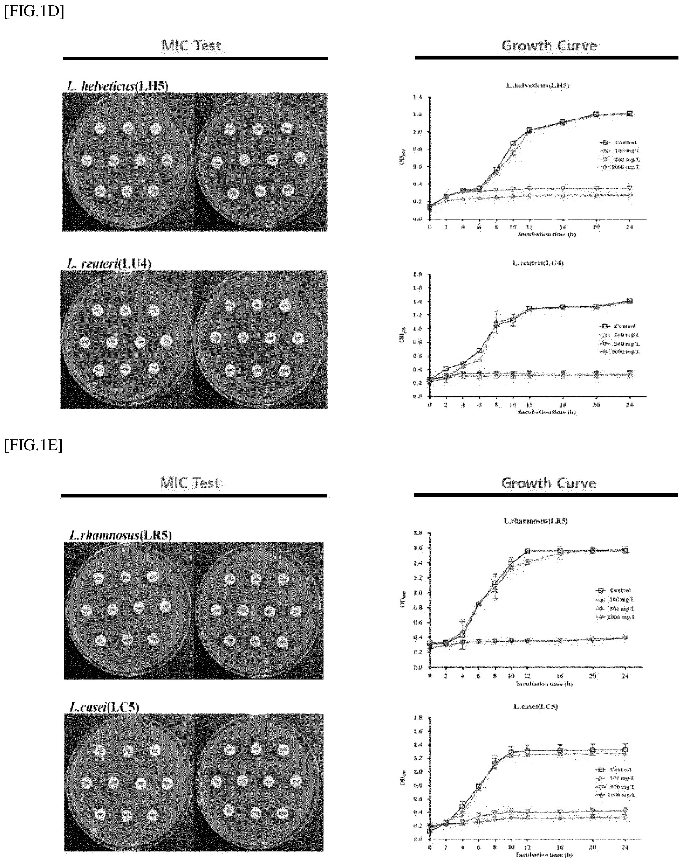

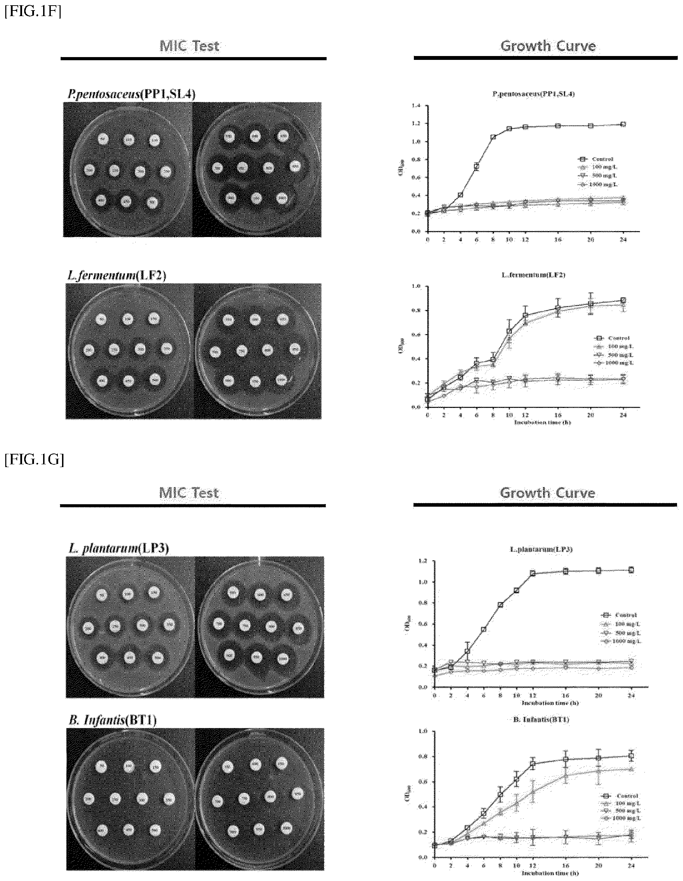

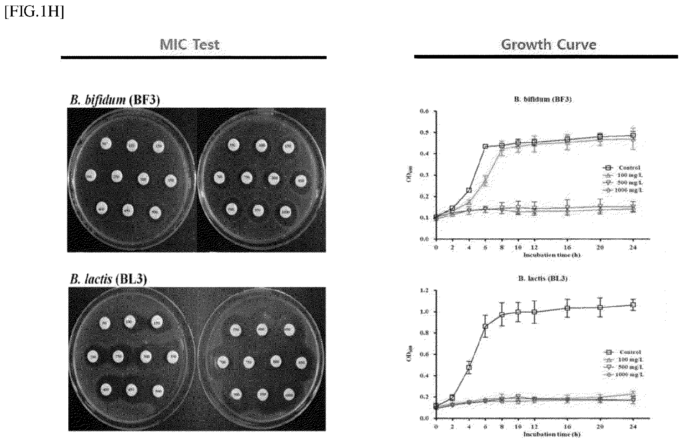

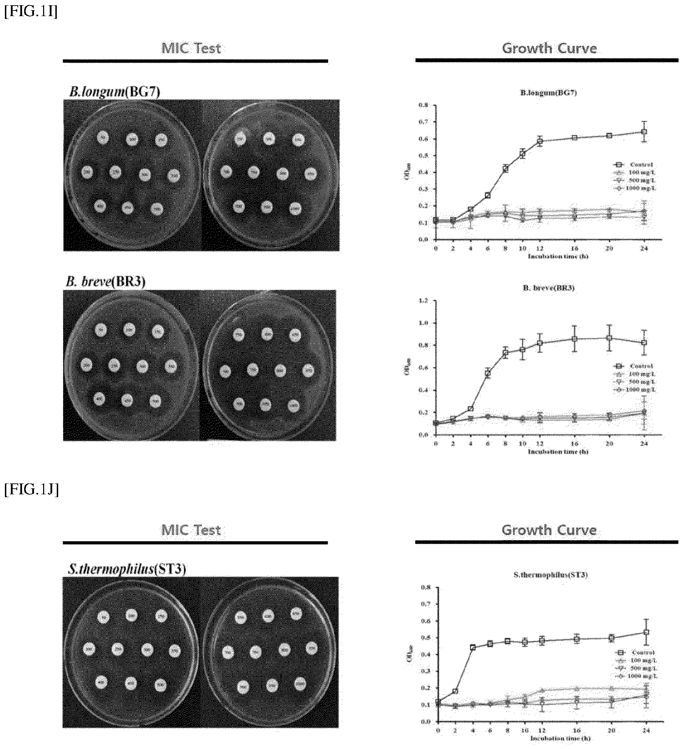

Table 2 shows the tolerance ability of each individual strain with respect to Cd, and according to cadmium tolerance ability results through antibacterial activity tests (MIC tests) of a total of 19 types, tolerance to cadmium of LA1, LGA, EF4, EFL2, SL6 > LPC5 > LH5 > LU4, BT1 > LR5, BF3 > LC5 > PP1 (SL4) > LF2, LP3, BL3, BG7, BR3, ST3 is shown in this stated order (in Table 1), and a growth curve pattern of each strain also showed a similar aspect to the cadmium concentration shown in the antibacterial activity tests (MIC tests) (see FIGS. 1A to 1J).

### Example 2. Results (in vitro) of evaluation of heavy metal removal capacity by probiotics with respect to cadmium (Cd)

After each strain was reacted with 50 mg/L of a cadmium solution for 1 hour, the residual cadmium concentration was measured. All strains were effective in removing cadmium, but strains LF2, SL6, LP3, LH5, LA1, and ST3 showed higher removal capacity that is greater than the total average value of 28.8 %.

With respect to the six strains (LF2, SL6, LP3, LH5, LA1, and ST3) selected from 50 mg/L of the cadmium solution, each additional removal capacity was measured in 5 mg/L of a cadmium solution and 100 mg/L of a cadmium solution.

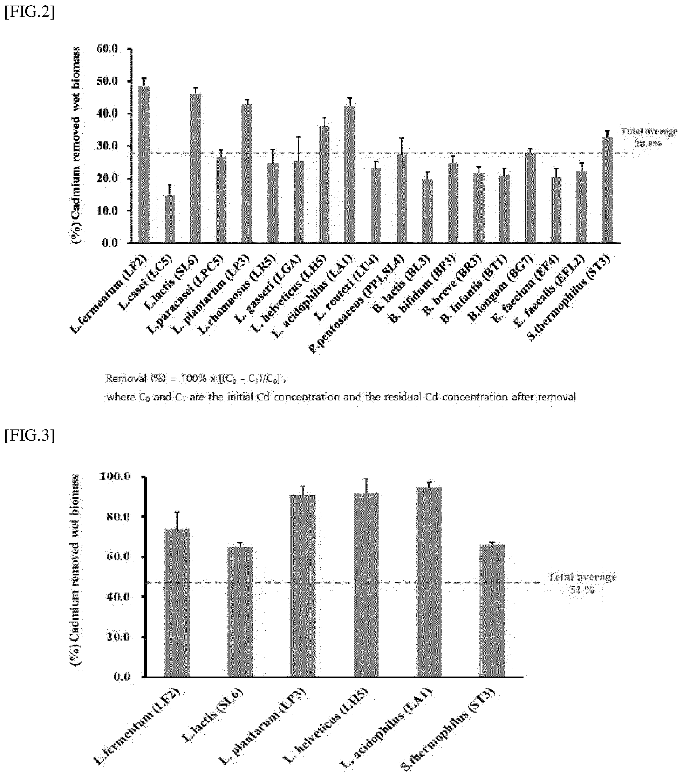

The average of the removal capacities in 5 mg/L of the cadmium solution was 51 %, and the removal capacity of each of the strains LP3, LA1, and LH5 among the six strains was 90 % or more. The average of the removal capacities in 100 mg/L of the cadmium solution was 21.6 %, and the removal capacity of each of the strains LP3, LA1, and LH5 among the six strains was 21.6 % or more (see FIGS. 2 to 4).

### Example 3. Results (in vitro) of evaluation of antibacterial activity tests (MIC tests) and bacterial growth by probiotics with respect to lead (Pb)

For each individual strain selected in Experimental Example 1, a heavy metal antibacterial activity test (MIC test) and bacterial growth were analyzed, and the tolerance ability is shown in Table 3 below.

**[Table 3]**

| Type of resistant | Strains | Cd MIC (mg/L) |
|---|---|---|
| | *L. acdidophilus* (LA1) | > 2,000 mg/L |
| | *L. gasseri* (LGA) | > 2,000 mg/L |
| | *E. faecium* (EF4) | > 2,000 mg/L |
| | *E. faecalis* (EFL2) | > 2,000 mg/L |
| | *L. lactis* (SL6) | > 2,000 mg/L |
| Lead-resistant | *L. paracasei* (LPC5) | > 2,000 mg/L |
| | *L. helveticus* (LH5) | > 2,000 mg/L |
| | *L. reureri* (LU4) | > 2,000 mg/L |
| | *B. infantis* (BT1) | > 2,000 mg/L |
| | *L. rhamnosus* (LR5) | > 2,000 mg/L |
| | *B. bifidum* (BF3) | > 2,000 mg/L |
| | *L. casei* (LC5) | > 2,000 mg/L |
| | *P. pentosaceus* (PP1,SL4) | > 2,000 mg/L |
| | *L. fermentum* (LF2) | > 2,000 mg/L |
| | *L. plantarum* (LP3) | > 2,000 mg/L |
| | *B. lactis* (BL3) | > 2,000 mg/L |
| | *B. longum* (BG7) | > 2,000 mg/L |
| | *B. breve* (BR3) | > 2,000 mg/L |
| | *S. thermophilus* (ST3) | > 2,000 mg/L |

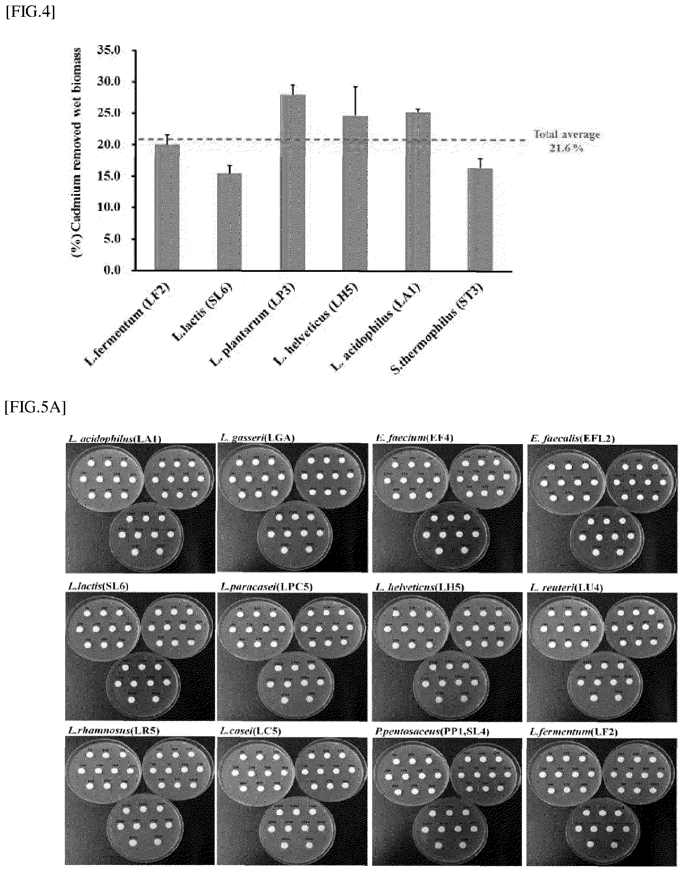

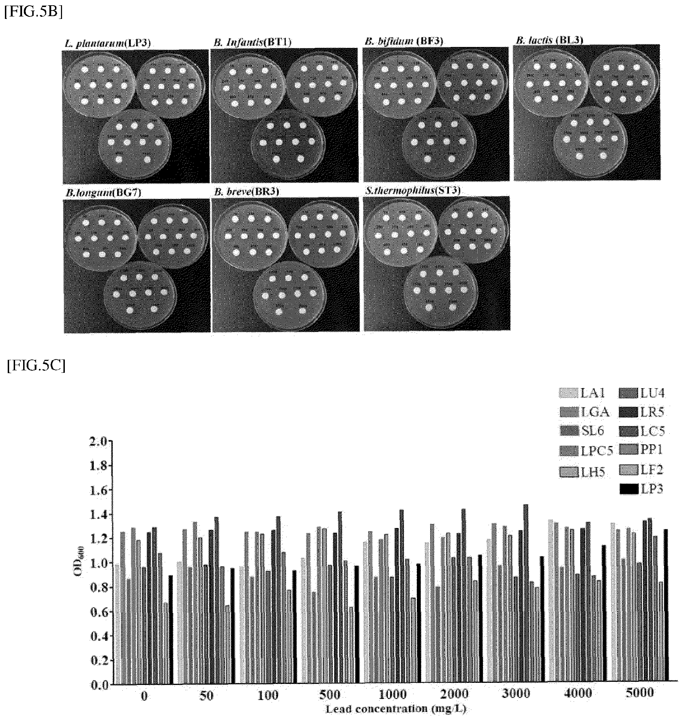

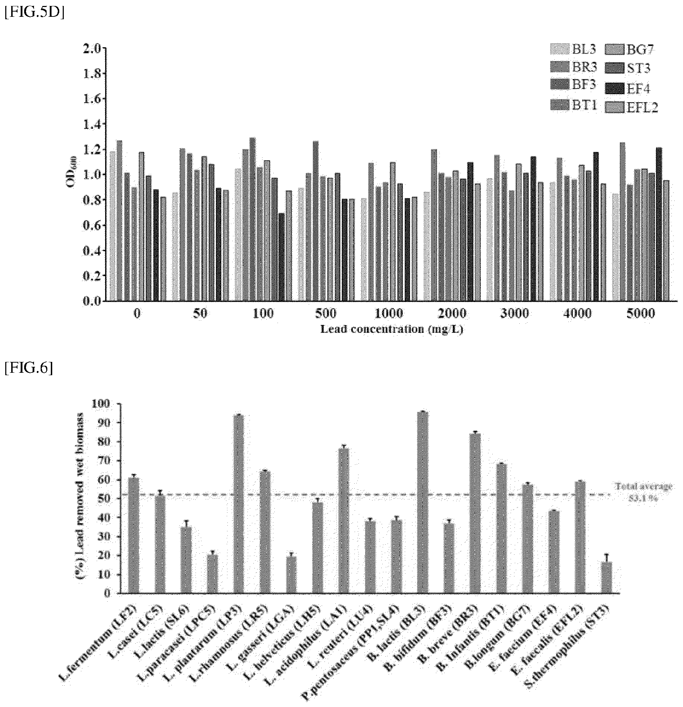

Table 3 shows the tolerance ability of each individual strain with respect to Pb, and showed a different aspect from the cadmium tolerance ability results through the antibacterial activity tests (MIC tests) of the total of 19 types. It was found that OD values measured after 24 hours of incubation in the MRS or BL broth showed a similar aspect to a control group, regardless of Pb concentrations (see FIGS. 5A to 5D).

### Example 4. Results (in vitro) of evaluation of heavy metal removal capacity by probiotics with respect to lead (Pb)

**After each strain was reacted with 50 mg/L of a lead solution for 1 hour, the residual lead concentration was measured.** It was found that each strain was effective in removing lead overall, but the strains had different degrees of removal efficacy. The total average of the strains as a whole had a removal efficacy of about 53 %, but among these, the strains LF2, LP3, LR3, LA1, BL3, BR3, BT1, BG7, and EFL2 were greater than or equal to the average, and in particular, LP3 and BL3 each showed a high removal efficacy of about 95 %.

With respect to the nine strains (LF2, LP3, LR5, LA1, BL3, BR3, BT1, BG7, and EFL2) selected from 50 mg/L of the lead solution, each additional removal capacity was measured in 5 mg/L of a lead solution and 100 mg/L of a lead solution.

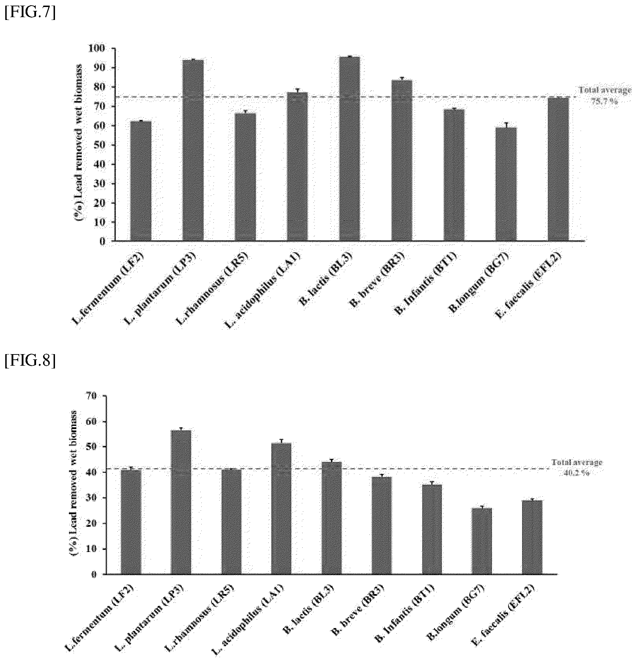

The average of the removal capacities in 5 mg/L of the lead solution was 75.7 %, and the removal capacity of each of the strains LP3, BL3, and BR3 among the nine strains was greater than or equal to the average. The average of the removal capacities in 100 mg/L of the lead solution was 40.2 %, and among the nine strains, LP3 and LA1 each showed the highest removal capacity (see FIGS. 6 to 8).

### Example 5. Results (in vitro) of evaluation of heavy metal removal capacity by probiotics with respect to lead (Pb)

Samples obtained by administering *Lactobacillus plantarum* and *Lactobacillus acidophilus* to 100 mg/L of a cadmium solution and 100 mg/L of a lead solution were observed morphologically through SEM imaging.

As a result of the SEM observation, it could be found that cadmium or lead particles were deposited and formed, through bright regions on the cell surface in each sample (see FIG.9).

### Example 6. Evaluation (in vivo) of heavy metal removal capacity by probiotics through body weights and liver weights

Body weights and liver weights, which were changed by probiotics, were evaluated through animal testing. It was found that the body weight and liver weight of the group administered with Cd were measured to be significantly less than those of groups administered with other substances, but the group administered with Cd was measured to have a similar weight to the Normal experimental group by administering probiotics (LA1+LP3). The body weight of the group administered with Pb was also measured to be significantly small, and the group administered with Pb was measured to have a similar weight to the Normal experimental group by administering probiotics (LA1+LP3), but there was no significant difference in tissue weight.

**[Table 4]**

| **Group** | **Weeks** | | | | |
|---|---|---|---|---|---|
| | **0 week** | **1 week** | **2 week** | **3 week** | **4 week** |
| Normal | 242.2 ± 7.3 | 318.3 ± 15.3 | 343.3 ± 12.5 | 372.3 ± 12.9 | 401.3 ± 15.3 |
| Probiotics | 246.3 ± 6.5 | 333.8 ± 7.5 | 343.5 ± 7.1 | 383.5 ± 8.6 | 413.6 ± 7.8 |
| Cd | 242.7 ± 5.4 | 269.5 ± 9.6 ^{b,d} | 311.2 ± 8.2b^{b,d} | 352.8 ± 6.0 ^{a,d} | 370.8 ± 6.2 ^{b,d} |
| Pb | 244.8 ± 7.9 | 319.2 ± 7.9 | 334.3 ± 7.0 | 364.7 ± 6.8 ^{d} | 385.6 ± 15.1 ^{c} |
| Cd + Probiotics | 245.2 ± 5.7 | 285.7 ± 12.2 ^{a,d} | 334.0 ± 4.7 ^{f} | 375.8 ± 6.4 | 393.1 ± 6.7 ^{c, f} |
| Pb + Probiotics | 242.3 ± 6.4 | 324.3 ± 7.1 | 341.7 ± 12.4 | 380.2 ± 10.0 | 412.0 ± 8.3 ^{g} |

### Example 7. Evaluation (in vivo) of heavy metal removal capacity by probiotics through excretion of heavy metal into feces

In the first week of Cd administration, the concentration of Cd in feces was measured to be significantly high in the experimental group administered together with probiotics (LA1+LP3). The amount of Cd in the feces of the experimental group administered with Cd alone was 65-89 µg/g fecal during four weeks of administration, whereas, the amount of Cd in the feces of the experimental group administered together with probiotics (LA1+LP3) was 85.3-156.4 µg/g fecal, and thus, it was found that, in the fourth week, there was about a twofold difference.

Due to the administration of probiotics (LA1+LP3) from the second week of administration, the amount of Cd in the feces resulted in about twice the excretion efficacy, compared to the administration of Cd alone. This was due to excretion of Cd accumulated in a body into feces by probiotics (LA1+LP3), and thus, it may be seen that probiotics (LA1+LP3) may reduce the amount of Cd accumulated in a body.

Meanwhile, in the first week of Pb administration, the concentration of Pb in feces was measured to be significantly high in the experimental group administered together with probiotics (LA1+LP3). The amount of Pb in the feces of the experimental group administered with Pb alone was 34-44 µg/g fecal during four weeks of administration, whereas, the amount of Pb in the feces of the experimental group administered together with probiotics (LA1+LP3) was 48-186 µg/g fecal, and thus, it was found that, in the fourth week, there was a difference of about 4.5 times.

Due to the administration of probiotics (LA1+LP3) from the second week of administration, the amount of Pb in the feces resulted in about 2.5 times the excretion efficacy, compared to the administration of Pb alone. This was due to excretion of Pb accumulated in the body into feces by probiotics (LA1+LP3), and thus, it may be seen that probiotics (LA1+LP3) may reduce the amount of Pb accumulated in a body.

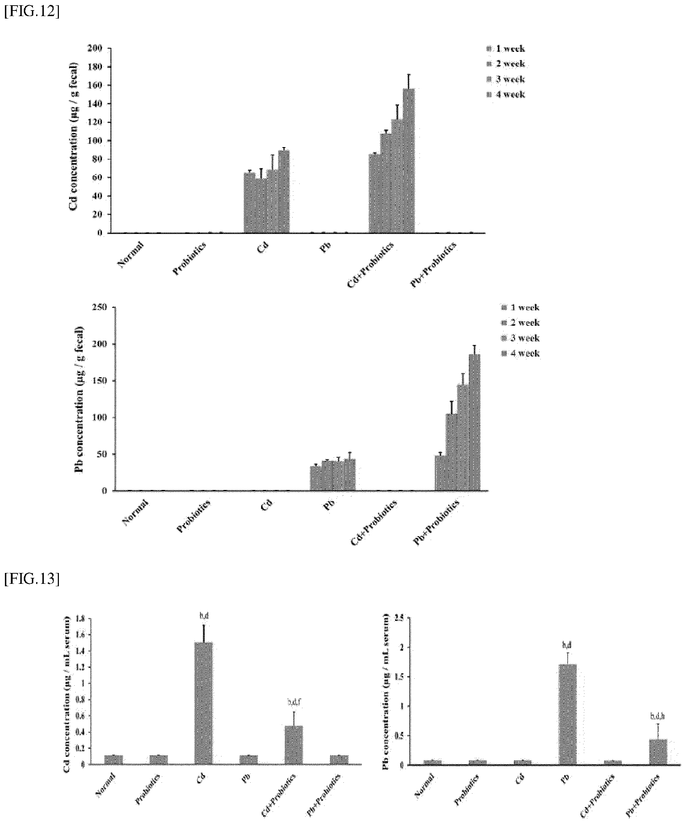

In addition, it was found that the administration of probiotics (LA1+L3) had a more effective expulsion effect in Pb compared to Cd (see FIG. 12).

**[Table 5]**

| **Group** | **Pb concentration (µg / g fecal)** | | | |
|---|---|---|---|---|
| | **1 week** | **2 week** | **3 week** | **4 week** |
| Normal | 0.38 ± 0.01 | 0.36 ± 0.05 | 0.38 ± 0.02 | 0.37 ± 0.03 |
| Probiotics | 0.44 ± 0.03 | 0.40 ± 0.08 | 0.41 ± 0.07 | 0.45 0.06 |
| Cd | 0.46 + 0.01 | 0.41 ± 0.07 | 0.40 ± 0.06 | 0.39 ± 0.06 |
| Pb | 33.99 ± 2.52 | 40..88 ± 1.77 | 40.27 ± 5.32 | 43.31 ± 8.55 |
| Cd + Probiotics | 0.38 ± 0.01 | 0.37 ± 0.04 | 0.37 ± 0.01 | 0.38 ± 0.04 |
| Pb + Probiotics | 48.13 ± 4.33 | 105.12 ± 1703 | 144.50 ± 15.09 | 186.52 ± 11.57 |

### Example 8. Evaluation (in vivo) of heavy metal removal capacity by probiotics by measuring amount of heavy metals in blood and tissues (liver, kidney)

**The administration of Cd and Pb resulted in accumulation in the blood, liver, and kidney, thereby showing insignificantly increased levels.** In numerical terms, a greater amount is accumulated in tissues than in blood.

It may be seen that the amount of heavy metals accumulated in the blood and tissues is significantly reduced due to the administration of probiotics (LA1+LP3). Specifically, Cd in the blood, Cd in the liver, and Cd in the kidney were respectively reduced by 68 %, 56 %, and 58 %, and Pb in the blood, Pb in the liver, and Pb in the kidney were respectively reduced by 73 %, 70 %, and 76 %.

As shown in the results measured in the feces, it was found that the heavy metals were attached to probiotics (LA1+LP3) and expelled to the outside of the body, and thus, absorption of the heavy metals into the body was relatively reduced (see FIG. 13).

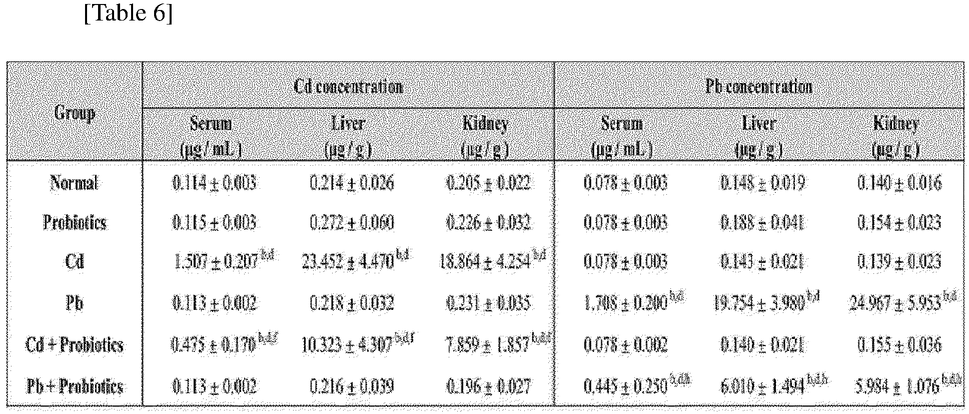

### Example 9. Evaluation (in vivo) of heavy metal removal capacity by probiotics by measuring hematological changes

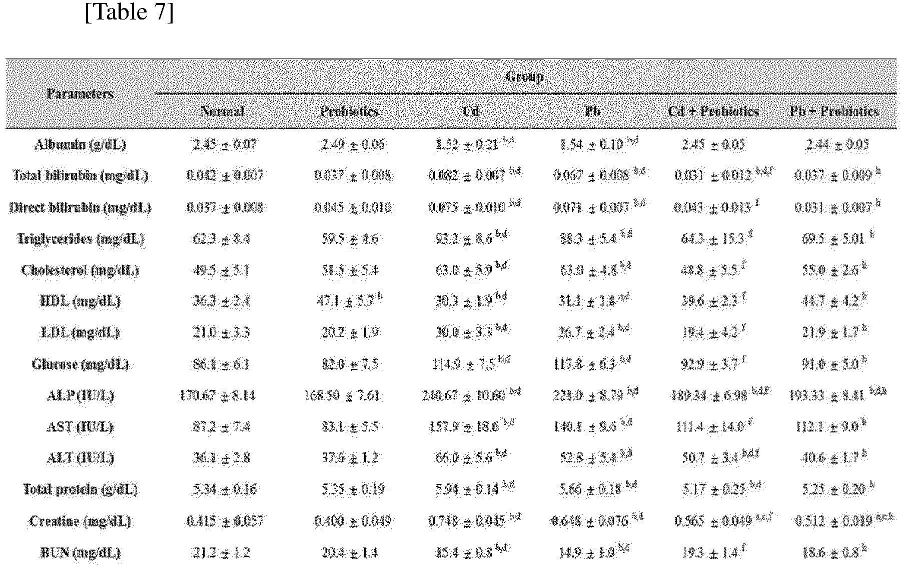

According to Table 7, 14 measured parameters were analyzed after 4 weeks of administration according to each experimental condition. As a result of the analysis, there were changes in liver and kidney status (toxicity) and lipid metabolism due to the administration of Cd or Pb. It was found that the administration of LA1+LP3 increased expulsion of Cd or Pb and thereby decreased absorption into the body, resulting in a parameter measurement value relatively closer to the Normal group (control group), and it was found that a HDL level significantly was increased by only the administration of LA1+LP3.

Hereinbefore, the specific parts of the present invention have been described in detail, and it is clear to those skilled in the art that these specific techniques are merely preferred embodiments and do not limit the scope of the present invention. Accordingly, the actual scope of the present invention will be defined by the appended claims and their equivalents.

## Claims

1. A composition for removing heavy metals, the composition comprising:
one or more bacteria selected from the group consisting of the genus Lactobacillus, the genus Bifidobacterium, the genus Enterococcus, the genus Pediococcus, and the genus Streptococcus.

2. The composition of claim 1, wherein bacteria of the genus *Lactobacillus* are one or more bacteria selected from the group consisting *of Lactobacillus acidophilus, Lactobacillus gasseri, Lactobacillus lactis, Lactobacillus paracasei, Lactobacillus helveticus, Lactobacillus helveticus reuteri, Lactobacillus rhamnosus, Lactobacillus casei*, La*ctobacillus fermentum,* and *Lactobacillus plantarum.*

3. The composition of claim 1, wherein bacteria of the genus *Bifidobacterium* are one or more bacteria selected from the group consisting of *Bifidobacterium infantis, Bifidobacterium bifidum, Bifidobacterium lactis, Bifidobacterium longum,* and *Bifidobacterium breve.*

4. The composition of claim 1, wherein bacteria the genus *Enterococcus* are one or more bacteria selected from the group consisting of *Enterococcus faecium* and *Enterococcus faecalis.*

5. The composition of claim 1, wherein bacteria of the genus *Pediococcus* are *Pediococcus pentosaceus.*

6. The composition of claim 1, wherein bacteria of the genus *Streptococcus* are *Streptococcus thermophilus.*

7. The composition of claim 1, wherein the composition has a tolerance to heavy metals.

8. The composition of claim 1, wherein the tolerance has one or more properties selected from minimum inhibitory concentrations (MICs), bacterial growth, and heavy metal removal or binding capacity.

9. The composition of claim 1, wherein the heavy metals are cationic heavy metals.

10. The composition of claim 9, wherein the cationic heavy metals are cadmium, and the bacteria has a cadmium removal capacity of 22 % or more in 100 mg/L of a cadmium solution.

11. The composition of claim 9, wherein the cationic heavy metals are lead, and the bacteria has a lead removal capacity of 22 % or more in 100 mg/L of a lead solution.

12. The composition of claim 1, wherein the composition has an effect of promoting expulsion of cationic heavy metals, and
bacteria of the genus *Lactobacillus* are one or more bacteria selected from the group consisting of *Lactobacillus acidophilus* and *Lactobacillus plantarum.*

13. A pharmaceutical composition for preventing or treating heavy metal poisoning, the pharmaceutical composition comprising the composition of claim 1.

14. A food composition comprising the composition of claim 1.

15. A cosmetic composition comprising the composition of claim 1.
